# EUROPÄISCHE PATENTSCHRIFT

(11) **EP 3 801 376 B1**
(45) Veröffentlichungstag und Bekanntmachung des Hinweises auf die Patenterteilung: **08.01.2025**
(21) Anmeldenummer: 19732737.2
(22) Anmeldetag: 23.05.2019
(51) Int. Cl.: A61F 2/00

(54) **VORRICHTUNG ZUM VERSCHLUSS VON KÖRPERÖFFNUNGEN MIT NEUTRALEM TRAGEKOMFORT**
DEVICE FOR SEALING BODILY ORIFICES, WITH NEUTRAL WEARING COMFORT
DISPOSITIF DE FERMETURE D'ORIFICES CORPORELS PRÉSENTANT UN CONFORT DE PORT NEUTRE

(30) Priorität: 28.05.2018 DE 102018004242
(43) Veröffentlichungstag der Anmeldung: 14.04.2021
(73) Patentinhaber: Advanced Medical Balloons GmbH, 68753 Waghäusel (DE)
(72) Erfinder: GÖBEL, Fred, 67346 Speyer (DE)
(74) Vertreter: Küchler, Stefan
(86) Internationale Anmeldenummer: PCT/IB2019/054278
(87) Internationale Veröffentlichungsnummer: WO 2019/229597

(56) Entgegenhaltungen:
- WO-A1-2009/144028
- WO-A2-2004/069057
- WO-A2-2008/124717
- DE-A1- 102012 003 034

## Beschreibung

Bei wachen und orientierten Patienten stellt sich bei der Anwendung von im oder am Körper verweilenden Katheterprodukten häufig das Problem einer möglichst irritationsfreien Positionierung, die dem Träger des Produktes bestmöglichen Tragekomfort und eine möglichst geringe Beeinträchtigung seiner körperlichen Bewegung gestattet. Dies gilt insbesondere für katheterartige Vorrichtungen, die zum dichtenden Verschluss von Körperöffnungen verwendet werden, aber auch für Katheter, die eine dichtende Verbindung vom Inneren des Körpers zur Körperoberfläche herstellen und beispielsweise für die geschlossen dichte Zuleitung und/oder Ableitung einer Substanz verwendet werden.

insbesondere bei körperlicher Arbeit oder auch sportlicher Aktivität kommt es zu vielseitig gerichteten Relativbewegungen zwischen der verschließenden Vorrichtung und den ihr unmittelbar exponierten Strukturen und Geweben. Der Grad der resultierenden Irritation kann vom Patienten bereits nach wenigen Minuten als derart störend empfunden werden, dass die Anwendung des Produktes beendet wird und von weiteren Anwendungen abgesehen wird.

Im Folgenden soll die Anforderung komfortablen Tragekomforts anhand einer primär dichtend wirkenden, katheterartigen Vorrichtung zum passageren, analen Verschluss bei ano-rektal inkontinenten Patienten beispielhaft erläutert werden.

Vorrichtungen zum intermittierenden Analverschluss basieren im Stand der Technik in der Regel auf im Rektum und/oder im Analkanal positionierte Materialien von schwammartiger Konsistenz. Durch Aufnahme von Darmsekret quillt der zunächst trockene, auf ein kleines Maß gepresste Verschluss-Korpus zu pilz- oder auch schalenartigen Formationen auf. Derartige Quellkörper werden vom Patienten häufig als fremdkörperartig empfunden. Insbesondere bei trans-anal, durch den Analkanal in das Rektum hindurchreichenden Verschlusskörpern kann es zu stark irritierenden Wahrnehmungen und auch zur Auslösung von Stuhldrang führen.

Neben Quellkörpern sind ebenfalls ballonartige, rektal und auch trans-anal positionierte Verschlusssysteme bekannt. Die dichtenden Ballonkomponenten dieser Vorrichtungen bestehen in der Regel aus elastisch dehnbaren Materialien, die sich bei der Befüllung durch den Anwender von einem bestimmten Fertigungsmaß ausgehend, auf ein bestimmtes, für die Funktion erforderliches Arbeitsmaß aufdehnen. Wegen der sich im elastisch expandierten Ballon einstellenden, relativ hohen Fülldrucke, wird die Mehrzahl der ballonbasierten Verschlüsse vom Patienten in Rektum und Anus als fremdkörperartig und störend wahrgenommen. Bekannte ballonbasierte Vorrichtungen zum Verschluss des Anus integrieren zudem ein schlauchartiges, relativ starr ausgeführtes Schlauchelement, dem der rektal ankernde und dichtende Ballonkörper aufsitzt. Um zu vermeiden, dass die Vorrichtung in das Rektum hineingleitet, kann der Schaftschlauch beispielsweise mit einem rechtwinkligen, T-förmigen Widerlager ergänzt werden, welches die Vorrichtung in der Analfalte fixiert und eine entsprechende Luxation aus der trans-analen Lage vermeidet. Schaftbasierte Ballon-Verschlüsse gehen zudem mit der grundsätzlichen Gefahr von Verletzungen des Darms einher, wenn es zu abrupten, zum Patienten hin gerichteten Auslenkungen der Vorrichtung kommt und der für die einfache Einführung des Verschlusskörpers erforderliche, rigide Schaftanteil der Vorrichtung eventuell die Wandung des Darms perforiert. Wegen der zuvor beschriebenen Nachteile haben sich bis heute in der Patientenversorgung keine derartigen Produkte etablieren können.

In ähnlicher Weise stellt sich die Problematik des bestmöglichen Tragekomforts bei gleichzeitig erhaltener Fähigkeit zur uneingeschränkten Bewegung des Patienten und einem im Verlauf der Anwendung kleinstmöglichem Traumarisiko bei Patienten, die mit einem künstlich angelegten Darmausgang (Stoma) versorgt sind. Insbesondere wegen der fortwährender Beugung, Aufrichtung und Torsion des Rumpfes können in fixer Weise auf einem Katheterschaft aufgebrachte Ballonelemente ein hohes Maß an subjektivem Missempfinden des Patienten verursachen.

Von den Trägern dauerhaft im oder am Körper platzierter verschlussartiger Vorrichtungen, sowie auch von zum Körper hin oder weg gerichteter Zuleitungen oder Drainagen werden daher neuartig konstruierte Vorrichtungen gewünscht, die optimalen Tragekomfort mit bestmöglicher Dichtungsleistung kombinieren, und auch dann gewährleisten, wenn sich die Person dauerhaft körperlich oder eventuell sogar sportlich betätigt. Gefordert sind Systeme, die eine hohe Anpassungsdynamik auch an extreme Körperlagen oder Auslenkungen aus einer Ruhelage haben und das Verletzungsrisiko in solchen Extremsituationen minimieren. Im idealen Falle ermöglichen derartige Systeme eine völlig druck- bzw. kraftneutrale Platzierung im Körper, die sich den im jeweiligen Organ herrschenden Kräften und Drucken selbständig angleicht, wodurch die Vorrichtungen vom Träger nach einer kurzen Phase der sensiblen Adaption gar nicht mehr wahrgenommen werden und vollständig neutral getragen werden können. Dennoch sollen die Systeme leicht einführbar und entnehmbar sein. Die Vorrichtungen sollen zudem einen möglichst einfachen, kostengünstigen Aufbau aufweisen.

Die vorliegende Erfindung schlägt zur Lösung dieser mehrfachen Anforderungen eine besondere Kombination von extrem dünnwandig ausgeführten, geleichzeitig dichtend und retinierend wirkenden Ballonfolienkörpern vor, die in neuartiger Art und Weise mit weichfolienartigen, adhäsiv aufgebrachten Haltestrukturen auf der Körperoberfläche kombiniert sind, die im Übergangsbereich zur des Ballonkorpus zur Körperoberfläche auf jede Art potentiell irritierender, als fest oder rigide empfundener Komponenten verzichtet. Die im Rahmen der Erfindung beschriebenen, membranartig dünnwandigen Folien-Materialien weisen dabei idealerweise eine hohe mechanische Stabilität bei anlastenden Druck- und Zugwirkungen auf, was eine für die Erfindung wesentliche Kombination von extrem leichter, neutral tragbarer Bauart und gleichzeitiger Formstabilität bei wechselnder mechanischer Belastung gestattet. Die eingesetzten Ballonfolien werden bevorzugt bereits bei der Herstellung auf ein Arbeitsmaß ausgeformt, dass für die dichtende und/oder retinierende Funktion erforderlich ist. Alternativ können die Ballonfolien dieses bestimmte Arbeitsmaß in einzelnen oder auch allen Abschnittsmaßen des Ballonkorpus bewusst und konzeptionell überschritten werden.

Die der Körperoberfläche bzw. Haut des Patienten adhäsiv fixiert anliegenden, ebenfalls sehr dünnwandigen Folien können optional mit einer gelartigen, beispielsweise auf Polyurethan basierten Materiallage beschichtet sein, wodurch sich die neutrale Tragecliarakteristik der das Gel tragenden Folie nicht verändert, aber die Handhabung, insbesondere das selbständige Aufbringen und Ablösen der dünnwandigen Klebefolie durch den Anwender erleichtert wird.

Kombinationen von mikrodünnen, vollständig ausgeformten, mechanisch stabilen Folienkörpern mit Widerlagerelementen sind bereits beispielsweise in DE 10 2005 021 081.3 order in WO2004/069057 beschrieben. Hier ist der Ballonkörper auf einem den Ballon tragenden Schaftelement aufgebracht. Durch eine besondere Invertierung bzw. zueinander hin versetzten Fixierung der Ballonenden auf dem Schaft wird eine axial gerichtete, dichtend wirkende Gegenrollung des im Körper platzierten Ballons zur inneren Mündung der Körperöffnung erzeugt. PCT/EP2004/008256 und PCT/EP2007/003099 beschreiben ebenfalls Vorrichtungen mit sehr dünnwandigen, vollständig ausgeformten Ballonkörpern, wobei hier ein Ende des Ballonkorpus vollständig oder nahezu vollständig durch das andere Ballonende hindurchgestülpt wird, und die beiden Ballonenden etwa auf gleicher Höhe an einem hülsenartigen Träger- und Abschlusselement aufgebracht sind. Das Hülsenelement ist hier extrakorporal oder auch im Zugangskanal zum Körper platziert und schließt die in sich rückgestülpte Ballonfigur in dichtender Weise als befüllbares Kompartiment ab. Ähnlich wie bei DE 10 2005 021 081.3 kommt es auch bei dieser Ausführung bei einer Beaufschlagung des Ballonkorpus mit Fülldruck zu einer axial gerichteten Rollbewegung des toroidalen Ballonkorpus zur inneren Mündung der Körperöffnung.

Das Hülsenelement stellt bei den in PCT/EP2004/008256 und PCT/EP2007/003099 beschriebenen Ausführungen die strukturelle Verbindung des Ballonkorpus zu einem extrakorporalen Kappenelement dar, im anderen Fall ist die Hülse mit einer schaftartigen Katheterkomponente verbunden, welche dem Anwender die Einführung der Vorrichtung erleichtert.

Die vorliegende Erfindung vermeidet sämtliche Komponenten fester oder rigider Konsistenz, wobei die Folienstruktur des intra-korporal platzierten Ballonkorpus direkt in die Folienstruktur der extrakorporalen Haltefläche übergeht bzw. ohne verbindende Funktionselemente mit potentiell irritierenden Eigenschaften erfolgt. Die erfindungsgemäße Vorrichtung besteht insbesondere im Bereich des trans-analen und des prä-analen Segmentes ausschließlich aus dünnwandigen Folienkomponenten. Die im Rahmen der Erfindung vorgeschlagenen Vorrichtungen sind im Tragezustand sämtlich schaftlos. Ein Ende der ausgeformten Ballonfolie wird bevorzugt durch den Ballonkorpus rückgestülpt und wird im Bereich des Übergangs zur Körperoberfläche oder im Bereich des trans-luminalen Zugangs zum Organ mit dem anderen Ballonende dicht schließend verbunden. Diese Form der Rückstülpung erzeugt ein zentrales, durchgängig offenes Lumen bzw. einen Zugangskanal zum jeweiligen Organ her. Der kanalartige Zugang kann beispielsweise für den Einschub einer retrahierbaren Einführhilfe genutzt werden. Der Kanal gestattet ferner die kontinuierliche Entgasung von Darmgas.

Unmittelbar an den Verbindungsbereich der beiden Enden der Ballonfolie schließt sich die Haltefolie bzw. deren propeller-, flügel- oder laschenartigen Ausziehungen für die Adhäsion an den Gesäßinnenseiten an. Die Haltefolie kann als separates Folienelement hergestellt werden, aber auch direkt, in eines der Enden des Ballonkorpus übergehend, an- bzw. ausgeformt werden.

Die Erfindung schlägt insbesondere die Kombination eines mikrodünnen Ballonkörpers aus durch Blasformung hergestellten, thermoplastischen Polyurethan-Ballorifolie vor, die wiederum mit einer aus thermoplastischem Polyurethan bestehenden Haltefolie verbunden ist. Die Verbindung kann durch Klebung oder Verschweißung hergestellt werden. Die Haltefolie wird bevorzugt durch Tiefziehung hergestellt und weist eine stutzen- oder konusartige zum Anus hin gerichtete oder auch in den Analkanal hineinreichende Ausziehung auf, die eine einfache, passgenaue Fügung an die konzentrischen Schlauchlagen des Ballonkorpus ermöglicht.

Weitere Merkmale, Eigenschaften, Vorteile und Wirkungen auf der Basis der Erfindung ergeben sich aus der folgenden Beschreibung sowia anhand der Zeichnung, worin bevorzugte Ausführungsformen der Erfindung offenbart sind. Hierbei zeigt:
- Abb.1: einen vollständig in sich rückgestülpten, schaftlosen Ballonkorpus mit einer für die extrakorporale Adhäsion an den Ballonkorpus angefügten Haltefolie, am Beispiel des natürlichen Darmausgangs;
- Abb. 2a: eine Vorrichtung entsprechend der Ausführung in Abb. 1, wobei das distale Ballonende mit einem dort fixierten, olivenförmigen Einführelement sowie einer retrahierbaren Einführhilfe ausgestattet ist;
- Abb. 2b: eine hinsichtlich ihres Aufbaus der Abb. 2a entsprechende Vorrichtung, wobei das distale Ballonende ein fingerlingartiges Einführelement integriert;
- Abb. 2c: eine Modifikation der Bauweise nach Abb. 2b, wobei der die Vorrichtung ebenfalls vom Finger des Anwenders aufgenommen und so digital eingeführt wird;
- Abb. 3a: den Übergang der Ballonfolie in die Haltefolie im Rahmen einer beispielhaften Ausführungsform;
- Abb. 3b: eine weitere Ausführungsform des Folienübergangs;
- Abb. 4: eine Kombination der Haltefolie mit einer gelartigen, die Haptik der Haltefolie modifizierenden Substanz;
- Abb. 5: eine besondere, propellerförmige Ausführungsform der Haltefolie für die Fixierung der Verschlussvorrichtung auf der peri-analen Haut der Gesäß-Innenflächen;
- Abb. 5a: besondere, auf der Haut der Gesäß-Innenflächen aufgeklebte Unterlagen, auf welche die propellerförmigen oder streifenförmigen Haltefolien der intermittierend gewechselten Verschluss-Vorrichtung reversibel lösbar, in adhäsiver Weise fixiert werden können;
- Abb. 5b: eine besondere Ausführungsform einer Einführhilfe zur Bestimmung der korrekten ano-rektalen Einführtiefe und zur erleichterten Aufklebung der lateralen Flügel bzw. Ausläufer auf die peri-anale Haut oder Unterlage;
- Abb. 6: eine entsprechende Ausführungsform für die intermittierende, verschließende Dichtung eines künstlichen Darmausgangs (Stoma); sowie
- Abb. 6a: eine kreis- bzw. reifenförmig ausgeschnittene, adhäsiv aufgebrachte Unterlage, beispielsweise für die Verwendung bei Ileo-Stomata.

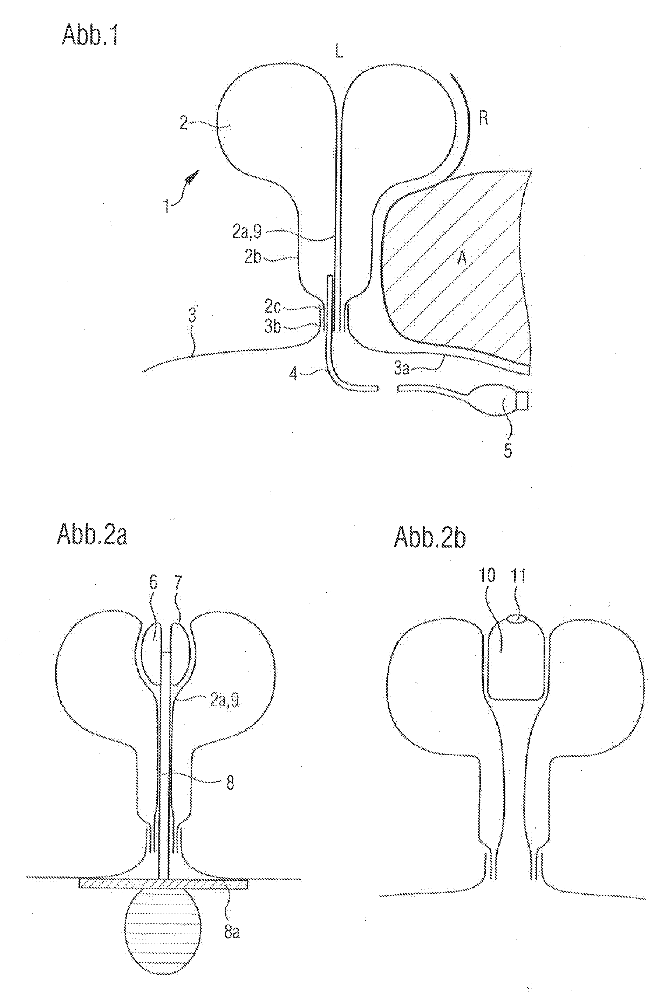

Abb. 1 zeigt eine erfindungsgemäße Vorrichtung 1, insbesondere in Form eines Analverschlusses, bestehend aus einem Ballonkorpus 2, dessen beide Ballonenden 2a und 2b zueinander parallel verlaufen und durch eine entsprechende Angleichung der jeweiligen endständigen Durchmesser im Bereich 2c miteinander dicht schließend, einen mit einem bevorzugt gasförmigen Medium befüllbaren Raum bildend, verbunden werden, sowie ferner bestehend aus einer adhäsiv wirkenden Haltefolie 3, die die Verschluss-Vorrichtung peri-anal fixiert auf den Gesäß-Innenseiten des Patienten fixiert und so eine ungewünschte Luxation der Vorrichtung aus der für die Erfindung chakteristischen, dichtend wirkenden, trans-analen Positionierung in das Rektum hinein vermeiden soll, wie sie ohne entsprechende peri-anal fixierende Komponenten, insbesondere beim Übergang von einer stehenden in eine sitzende Haltung des Patienten, regelmäßig beobachtet werden.

An das durch den Bereich 2c definierte proximale Ende des befüllbaren Korpus schließt sich direkt und ohne verbindendes oder vermittelndes Element die Haltefolie 3 an. Die Haltefolie verfügt über eine zum Patienten hin gerichtete adhäsive Fläche 3a. Sie weist eine stutzenartige Tiefziehung 3b auf, die kongruent mit der Innen- oder Außenfläche des Bereiches 2c überlappt und durch beispielsweise Klebung oder Schweißung mit dieser unmittelbar verbunden ist. Im Fügebereich 2c der beiden Ballonenden ist zwischen den beiden konzentrischen Folien- oder auch Schlauchlagen in dichtender Weise eine den Ballonkorpus befüllende Schlauchzuleitung 4 eingefügt, an deren Ende ein Pilotballon 5 mit integriertem Füllventil angeschlossen ist.

Die Positionierung des Verschlusses im bzw. am Patienten erfolgt derart, dass die beiden konzentrisch verlaufenden Ballonenden 2a und 2b durch den Anus A laufen und der toroidale Ballonkorpus 2 dem Rektumboden R aufliegt. Das sich durch die beiden Ballonenden ergebende trans-anale Segment des Ballonkorpus ist im Durchmesser relativ zum rektal positionierten Torus verjüngt. Der Durchmesser des Ballonendes 2b wird bevorzugt mit ca. 15 bis 30 mm ausgeführt, besonders bevorzugt mit 20 bis 25mm. Der Durchmesser des innenliegenden Ballonendes 2a liegt bei ca. 15 mm und lässt im idealen Falle die Aufnahme der Vorrichtung auf einem Finger des Anwenders bzw. die digitale trans-anale Einführung des Ballonkorpus zu. Alternativ zu einem aus dem Ballon direkt hervorgehenden bzw. in einem Stück ausgeformten Ballonende 2a kann das zentrale Lumen L, welches das distale Ballonende mit dem proximalen Ballonende durchgängig verbindet, auch durch ein separat hergestelltes Schlauchsegment 9 hergestellt werden

Der Ballonkorpus wird in situ bevorzugt inkomplett gefüllt. Das verwendete Füllvolumen beträgt etwa 80% des Volumens des frei ausgeformten bzw. frei entfalteten Ballonkorpus. Der Ballon geht so in einen für die Funktion der Vorrichtung besonders vorteilhaften, schlaffen, spannungslosen Füllzustand über. Der Ballonkorpus nimmt im schlaffen Zustand die jeweilig herrschende rektale Kraft auf, überträgt selbst aber keine permanent wirkende Kraft auf die ihm anliegenden anatomischen Strukturen und Gewebe. Der im Ballon herrschende Fülldruck verhält sich zu den rektal bzw. abdominal wirkenden Drucken nahezu zeitsynchron, und gewährleistet so eine durchgängig wirkende, zur Schleimhaut des Analkanals gerichtete, dichtende Entfaltung des trans-anal positionierten, äußeren Ballonendes 2b. Währenddessen kollabiert, bedingt durch die Kraftwirkung des im Ballonkorpus herrschenden Fülldrucks, das zentrale, durch das innere Ballonende 2a formierte Lumen L, eine entsprechende Verformbarkeit vorausgesetzt, in einer das Lumen nahezu verschließenden Weise.

Abb. 2a zeigt eine Abb. 1 entsprechende Ausführungsform der Vorrichtung, wobei in die distale bzw. vordere Öffnung des Lumens L ein olivenartiges Einführelement 6 fest eingefügt, welches über einen zentralen Kanal 7 verfügt, in dessen proximales Ende eine aus der Olive retrahierbare, stabartige Einführhilfe 8 eingesteckt ist. Die Innenlage 2a der Vorrichtung kann alternativ als ein separat ausgeformtes Schlauchfolienelement 9 hergestellt werden, das sich an die Olive nach proximal anschließt, und sich im Bereich 2c mit der Außenlage 2b dicht schließend verbindet.

Die Einführhilfe 8 kann mit einem diskoiden und/oder tellerartigen Element 8a versehen sein, welches dem Anwender beim Einführen der Vorrichtung durch mechanischen Anschlag des Diskus am Anus die korrekte Einführtiefe anzeigt. Dem Element 8a liegt die Haltefolie 3 dabei distal, also zum Patienten gerichtet, auf.

Abb. 2b zeigt eine entsprechende Ausführung zu Abb. 2a, wobei in die distale Öffnung des Lumens L ein fingerling-artiges Element 10 in fest verbundener Weise eingefügt ist, in das das Endglied des einführenden Fingers eingeführt werden kann. Zur Dekompression von Darmgas ist der Fingerling 10 am distalen Ende bevorzugt mit einer Öffnung 11 versehen. Die Öffnung kann mit einem gasdurchlässigen, aber flüssigkeitsabweisenden Vlies versehenen sein.

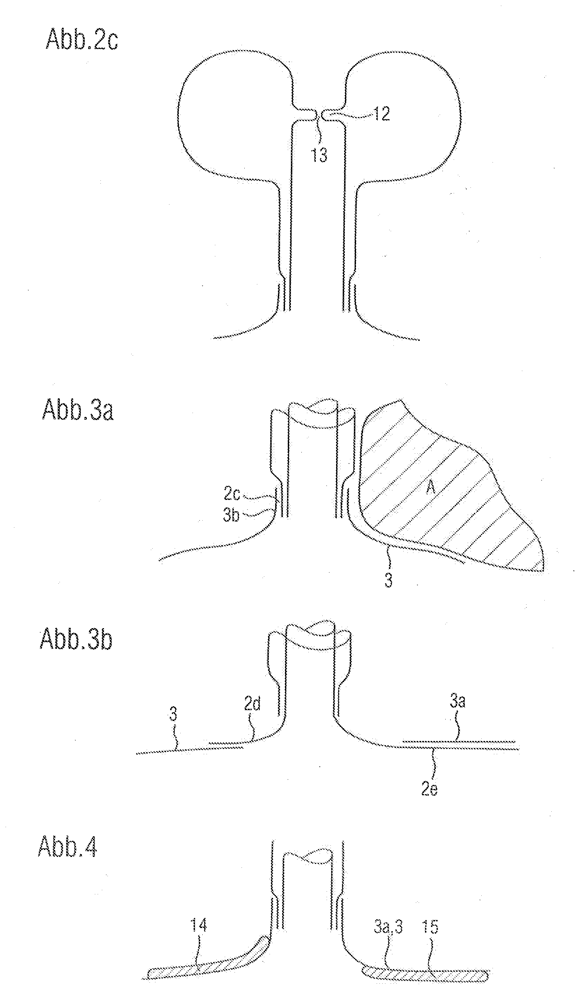

Abb. 2c zeigt eine entsprechende Ausführung zu Abb. 1 und Abb. 2b, wobei im distalen Bereich des Lumens L in die Innenlage bzw. das innere Ballonende 2a eine Engstelle 12 eingeformt ist, welche ebenfalls das Einführen eines die Vorrichtung aufnehmenden Fingers erlaubt. Das Lumen 13 im Bereich der Engstelle sorgt für kontinuierlichen Auslass von Darmgas.

Abb. 3a zeigt den Übergang der Folienenden des Ballonkorpus in die prä-anal haltende Ballonfolie 3 im Detail. Im Bereich 2c liegt der stutzen- bzw. trichterartige Ansatz 3b der Haltefolie von außen oder von innen an. Der Übergangsbereich ist vorzugsweise im äußeren Drittel des Analkanals A platziert. Die besonders druckempfindliche Rosette des Anus ist so lediglich der Haltefolie 3, und nicht dem Fügebereich der Ballonenden mit der Haltefolie exponiert. Die stutzenartige Ausziehung 3b der Haltefolie kann bei der Herstellung z.B. durch eine entsprechende Tiefziehungsmethode in die Folie hinein geformt sein.

Abb. 3b zeigt weitere Ausführungsoptionen des Übergangs von Ballon- und Haltefolie. So kann am proximalen Ende des inneren Ballonendes 2a eine flanschartige Erweiterung 2d ausgeformt sein, an die wiederum die Haltefolie 3 angeflanscht ist. Der Flansch 2d sollte dabei einen Durchmesser von ca. 4 bis 5 cm haben, um zu gewährleisten, dass die doppellagige Verbindungsfläche des Verbindungsbereichs in ausreichendem Abstand von der Rosette positioniert ist. Alternativ kann das Flansch 2d derart groß im Durchmesser ausgeformt sein, dass es keiner weiteren Haltefolie 3 mehr bedarf und das Adhäsiv 3a dann direkt auf der zum Patienten gerichteten Innenseite des Flansches aufgebracht werden kann. Die mit Adhäsiv beschichtete Klebefläche sollte mindestens einen Abstand von 15 mm zum Rande der analen Rosette haben.

Abb. 4 zeigt eine besondere Kombination der Haltefolie 3 mit einer adhäsiv wirkenden, gelartigen Substanz, die entweder auf der dem Patienten zugewandten Seite 14 aufgebracht sein kann, oder auf der vom Patienten abgewandten Seite 15. Weist das Gel zum Patienten hin, können beispielsweise mit Polyurethan-basierten Gelen Klebeeigenschaften erreicht werden, die für die Adhäsion der Vorrichtung auf der Haut des Patienten ausreichen. Der vorteilhafte Effekt der Gele besteht in deren gewebenahe Beschaffenheit und einer guten, wenig irritierenden Hautverträglichkeit. Wird das Gel auf der dem Patienten abgewandten Seite aufgetragen, kann durch eine entsprechende gelartige Fläche oder Teilfläche sowohl das manuelle Aufbringen, als auch die Lösung bzw. Entfernung der Haltefolie von der Haut durch den Patienten erleichtert werden. Das Gel ist zu diesem Zweck auf der dem Patienten abgewandten Seite mit einer geeigneten Beschichtung oder Einhausung 15a versehen, die die natürliche Adhäsion weicher PUR-Gele nimmt.

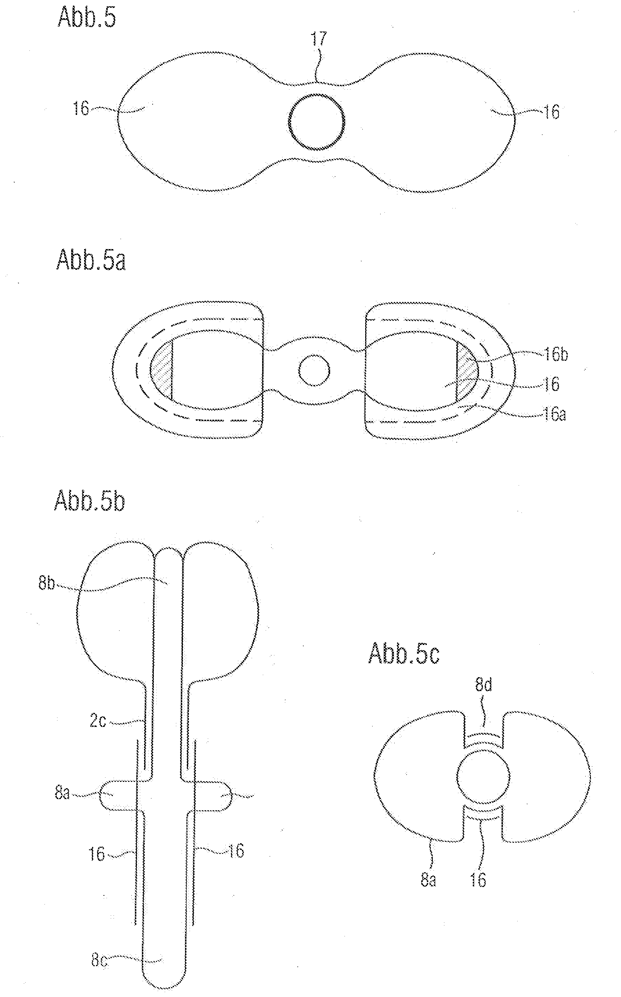

Abb. 5 zeigt eine für den gering irritierenden Tragekomfort besonders vorteilhaft zugeschnittene, propellerartige Haltefläche 16, deren beide Flügel jeweils auf die an die Rosette angrenzende peri-anale Haut der Gesäßbacken aufgeklebt werden. Die mittige, besonders schlanke Portion 17 liegt unmittelbar vor dem Anus. Durch die Verschlankung von den lateralen Flügeln hin zur mittigen Portion 17, können scherende bzw. torquierende Wirkungen der Gesäßpartie beim Laufen auf den anal bzw. trans-anal positionierten Anteil der Verschluss-Vorrichtung weitgehend verhindert werden. Die Entkopplung glutealer Bewegungen bzw. von Massenbewegungen des Körpers vom retinierend und dichtenden Ballonkorpus ist für ein möglichst neutrales Trageempfinden des Anwenders entscheidend.

Abb. 5a geht insbesondere auf den Komfort bei der Lösung der adhäsiven Haltefolie vom Patienten ein. Um ein schmerzhaftes Ablösen der klebenden Folie von der empfindlichen peri-analen Haut zu vermeiden, werden auf beiden peri-analen, glutealen Hautpartien flächige Unterlagen 16a aufgeklebt, die aus einem bevorzugt transparenten Folienmaterial bestehen, das für eine dauerhafte Haut-Exposition geeignet ist. Die Erfindung bevorzugt für die Unterlagen adhäsive Folien mit Eigenschaften, wie sie beispielsweise vom Hersteller 3M Health Care in Art des Produktes Tegaderm Absorbent angeboten werden. Das Produkt weist ein zentrales, flächiges Teilstück auf, dass zum umgebenden Saum eine prominente Wandungsstärke und eine erhöhte Rigidität auf, und ermöglicht so die taktile Wahrnehmung des für die Anheftung der Haltefolie besonders günstigen Bereiches der Unterlage. Hat der Anwender beim Einführen und Fixieren des Ballon-Verschlusses direkte Sicht auf den peri-analen Bereich, beispielsweise durch Verwendung eines Spiegels, kann die relative Positionierung der Haltefolie zur Unterlage ferner durch eine Einfärbung der Unterlage 16a im Bereich dieser günstigen Positionierung erleichtert werden.

Die beschriebene adhäsive Folienunterlage 16a besteht vorzugsweise aus hydrokolloidalem, atmungsaktivem Material, ist wasserfest und erlaubt sowohl regelmäßiges Duschen und eine normale Anal-Toilette. Reaktive Veränderungen der Haut können durch die Transparenz des Materials ggf. gut erkannt werden.

Auf die beiden Unterlagen 16a, die über mehrere Tage, bis hin zu einer Woche peri-anal positioniert verbleiben können, werden die propeller- oder auch streifenförmigen Haltefolien 16 aufgeklebt. Die adhäsive Wirkung der Unterlage 16a zur Haut des Patienten ist dabei größer als die Adhäsion der Haltefolie zur Unterlage. Ferner sollte die Kontur der Unterlage 16a die Kontur der fixierenden Ausläufer 16 der Haltefolie 3 überragen, um so zu gewährleisten, dass die Haltefolie mit einer gewissen Sicherheit in der Anwendung vom Patienten auf der Unterlage erfolgt. Für eine derartige Technologie eignen sich als fixierendes Adhäsiv zwischen Haltefolie 3 und Unterlage insbesondere sehr dünn-lagig aufgetragene, Polyurethan basierte Gele, die einen etwa "Post-it" artigen Löse-Effekt von der unterlegten Folie erlauben. Zur Vermeidung der beschriebenen Luxation der Verschlussvorrichtung aus der trans-analen Position in das Rektum ist eine insgesamt moderat wirkende Haftung zur beschriebenen Folienunterlage ausreichend.

Zur Erleichterung der Ablösung der propeller- oder streifenförmigen Haltefolie von der Unterlage sind die Enden der Folienausläufer 16 mit einem nicht klebenden Folienbereich 16b versehen, der vom Anwender leicht gegriffen werden kann.

Abb. 5b zeigt eine besondere Ausführung einer erfindungsgemäßen Verschluss-Vorrichtung, wobei der rektal und trans-anal zu positionierende Verschlusskorpus auf einem fingerartigen Element 3b aufgebracht ist, und die Einführtiefe dem Anwender durch eine flanschartige, tellerartigen oder diskoiden Anschlag 8a vorgegeben wird. Nach proximal, vom Patienten weggerichtet, geht das fingerartige Element in einen Haltegriff 8c über, der die manuelle Einführung in den Anus erleichtert. Der Anschlag verfügt über zwei einander gegenüberliegende Aussparungen 3d, in welche die auf die Gesäßbacken aufzuklebenden Haltefolien 16 parallel zum Haltegriff eingelegt werden. Die einführende Hand greift somit den Haltegriff samt Haltefolien, wodurch die Haltefolien die anale Einführung der Vorrichtung nicht behindern. Die Haltefolien 16 werden nach erfolgtem Anschlag des die Einführhilfe bestimmenden Diskus am Anus aus den Aussparungen 8d herausgeklappt und nach Entfernung bzw. Abziehen einer Schutzfolie auf die Gesäßbacken geklebt. Das fingerartige Element 8b wird dann aus dem zentralen Lumen L des Ballonkorpus herausgezogen und entfernt.

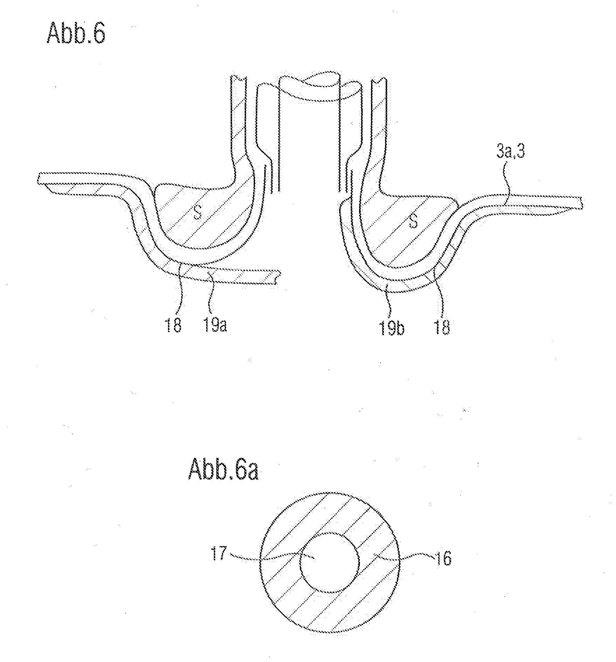

Abb. 6 zeigt eine besondere Ausführung der Haltefolie 3 für den Verschluss künstlicher Darmausgänge, insbesondere von sogenannten Ileostomata, die eine direkte Verbindung des Dünndarms eines Patienten zur abdominalen Hautoberfläche herstellen. Die Haltefolie 3 bzw. 18 ist dabei glockenartig vertieft ausgezogen. Die Vertiefung nimmt das auf die Bauchwand umgeschlagene und dort verwachsene Darmsegment S des Stomas auf. Die Außenseite der Folie 3 kann dabei von einer schützenden Gel-Lage 19 umgeben sein, die sich optional kappen- oder domartig 19a über das äußere Stoma legt, oder der glockenförmigen Kontur 19b der Folie angeschmiegt folgt.

Abb. 6a zeigt eine stoma-gerechte Variante einer Unterlage 16a, die die Lösung der Verschluss-Vorrichtung von der peri-stomalen Haut erleichtert. Sie ist in diesem Falle zirkulär ausgeschnitten und besteht bevorzugt aus sehr dünnwandiger, atmungsaktiver, wasserfester und abwaschbarer Folie, analog zur in Figur 5a beschriebenen Unterlage. Die Unterlage gestattet es den im Stoma positionierten Verschluss-Körper atraumatisch und schmerzfrei von der peri-analen Haut abzulösen. Die Unterlage wird in verschiedenen zirkularen Öffnungsdurchmessern 16c konfektioniert. Sie verfügt ebenfalls über einen nicht-adhäsiven Randabschnitt 16b, der vom Patienten sicher gegriffen werden kann und das Abziehen der Folie von der Haut erlaubt.

Der Ballonkorpus wird bevorzugt mit einem bestimmten, vorgegebenen LuftVolumen befüllt. Hierzu verwendet der Anwender beispielsweise eine herkömmliche Spritze, die mit einer entsprechenden Volumenmarkierung versehen ist. Das Volumen für die Befüllung des Ballonkorpus ist derart bemessen, dass sich der Ballon nur partiell füllt, also in einem schlaffen, spannungslosen Zustand verbleibt. In situ schmiegt sich der so befüllte Ballon der individuellen anorektalen Anatomie des Patienten in optimal dichtender Weise an. Der sich jeweils im Ballon einstellende Fülldruck entspricht in Näherung dem jeweilig herrschenden rektalen Druck. Dies wird in der Regel bei Füllvolumina erreicht die etwa 70 bis 80 % des frei ausgeformten Ballonkorpus der anwendungsbereiten Vorrichtung entsprechen.

Alternativ kann die Verschlussvorrichtung auch druckkontrolliert mit beispielsweise einem üblichen Hand-Pumpmanometer eingestellt werden. Die für die Verschlussfunktion erforderlichen Drucke sind dann individuell zu ermitteln.

### Bezugszeichenliste

| | | | |
|---|---|---|---|
| 1 | Vorrichtung | 16 | Haltefläche |
| 2 | Ballonkorpus | 16a | Unterlage |
| 2a | Ballonende | 16b | Folienbereich |
| 2b | Ballonende | 16c | Öffnungsdurchmesser |
| 2c | Bereich, Fügebereich | 17 | Portion |
| 2d | flanschartige Erweiterung | 18 | Haltefolie |
| 3 | Halteansatz | 19 | Gel-Lage |
| 3a | adhäsive Fläche | 19a | Dom, Kappe |
| 3b | trichterartiger Ansatz | 19b | glockenförmige Kontur |
| 4 | Schlauchzuleitung | A | Anus, Analkanal |
| 5 | Pilotballon | L | Lumen |
| 6 | Einführelement | R | Rektumboden |
| 7 | zentraler Kanal | S | Darmsegment |
| 8 | Einführhilfe | | |
| 8a | tellerartiges Element | | |
| 8b | fingerartiges Element | | |
| 8c | Haltegriff | | |
| 8d | Aussparung | | |
| 9 | Schlauchsegment | | |
| 10 | Fingerling | | |
| 11 | Öffnung | | |
| 12 | Engstelle | | |
| 13 | Lumen | | |
| 14 | Seite | | |
| 15 | Seite | | |

## Patentansprüche

1. Vorrichtung (1) zum minimal irritierenden, optimal organverträglichen Verschluss eines im Bereich der Körperoberfläche als Ostium mündenden Lumens (L), insbesondere eines natürlichen oder künstlichen Darmausgangs (A) eines Patienten, umfassend einen sehr dünnwandigen, von außen befüllbaren Ballonkorpus (2) aus einem weichfolienartigen Material geringer Volumendehnbarkeit, der wenigstens einen transostialen Bereich aufweist sowie ein zentrales Lumen, das sich vom distalen bis zum proximalen Ende der Vorrichtung (1) erstreckt und der Aufnahme einer Einführhilfe dient und/oder den Auslass von Darmgas erlaubt, **dadurch gekennzeichnet, dass**
a) das zentrale Lumen innerhalb des Ballonkorpus (2) nicht von einem Schaft umschlossen ist, so dass rigide, potentiell irritierend wirkende Komponenten im Bereich des Ostiums sowie in einem ggf. anschließenden, extrakorporalen Bereich gänzlich vermieden sind, und dass
b) sich an das proximale Ende des Ballonkorpus (2) wenigstens ein flächiger, mit einem hautverträglichen Adhäsiv beschichteter, weichfolienartiger Halteansatz (3) anschließt, welcher eine direkte Fixierung der Vorrichtung (1) auf der Haut des Patienten erlaubt.

2. Vorrichtung (1) nach Anspruch 1, **dadurch gekennzeichnet, dass** der Ballonkorpus (2) bei der Herstellung vollständig auf sein erforderliches Maß ausgeformt ist.

3. Vorrichtung (1) nach Anspruch 1 oder 2, **dadurch gekennzeichnet, dass** der Hohlraum des Ballons (2) zu dem umschlossenen Lumen hin durch eine Lage aus einem weichfolienartigen Material abgeschlossen ist, insbesondere wobei die den Hohlraum des Ballons (2) zu dem umschlossenen Lumen hin abgrenzende Lage aus einem weichfolienartigen Material mit der Außenhülle das Ballonkorpus (2) verbunden oder integral hergestellt ist.

4. Vorrichtung (1) nach einem der vorhergehenden Ansprüche, **dadurch gekennzeichnet, dass** zur Herstellung des Ballonkorpus (2) ein Ende (2a) eines zunächst schlauchförmigen Ballonrohlings nach innen umgestülpt und durch das andere Ende (2b) des Ballonrohlings hindurchgeführt ist, vorzugsweise wobei die beiden, ineinander geführten Ballonenden (2a,2b) dicht schließend miteinander verbunden sind, insbesondere im transostialen oder im transluminalen Bereich oder im unmittelbar angrenzenden, extrakorporalen Bereich, um dadurch einen von außen befüllbaren Raum herzustellen, insbesondere wobei die beiden, ineinander geführten Ballonenden (2a,2b) unmittelbar miteinander verbunden sind, also nicht über eine dazwischen eingefügte Hülse oder einen anderen Abstandhalter.

5. Vorrichtung (1) nach einem der vorhergehenden Ansprüche, **dadurch gekennzeichnet, dass** ggf. vorhandene, extrakorporale Bereiche des Ballonkorpus (2) gegenüber dem transostialen Ballonabschnitt nicht radial erweitert sind.

6. Vorrichtung (1) nach einem der vorhergehenden Ansprüche, **dadurch gekennzeichnet, dass** der Ballonkorpus (2) durch Blasformen hergestellt ist, insbesondere durch Blasformen aus einer thermoplastischen Polyurethanfolie.

7. Vorrichtung (1) nach einem der vorhergehenden Ansprüche, **dadurch gekennzeichnet, dass** der Halteansatz (3) aus einer dünnen, vorzugsweise membranartigen Weichfolie besteht, und/oder aus thermoplastischem Polyurethan besteht, und/oder durch Tiefziehen hergestellt ist.

8. Vorrichtung (1) nach einem der vorhergehenden Ansprüche, **dadurch gekennzeichnet, dass** der Halteansatz (3) mit einer gelartigen, beispielsweise auf Polyurethan basierten Materiallage beschichtet ist.

9. Vorrichtung (1) nach einem der vorhergehenden Ansprüche, **dadurch gekennzeichnet, dass** der Halteansatz (3) einen oder mehrere, von dem proximalen Ballonende (2a,2b) etwa radial weg ragende Laschen oder Flügel aufweist, insbesondere eine laschen-, flügel- oder propellerartige Grundfläche aufweist.

10. Vorrichtung (1) nach einem der vorhergehenden Ansprüche, **dadurch gekennzeichnet, dass** der Halteansatz (3) eine symmetrische Grundfläche aufweist, insbesondere mit zwei einander etwa diametral gegenüberliegenden Fortsätzen (16).

11. Vorrichtung (1) nach einem der vorhergehenden Ansprüche, **dadurch gekennzeichnet, dass** sich der Halteansatz (3) unmittelbar an den Verbindungsbereich (2c) der beiden Enden (2a,2b) der Ballonfolie anschließt.

12. Vorrichtung (1) nach einem der vorhergehenden Ansprüche, **dadurch gekennzeichnet, dass** der Halteansatz (3) in eines der Enden (2a.2b) des Ballonkorpus (2) übergehend an- bzw. ausgeformt ist.

13. Vorrichtung (1) nach einem der vorhergehenden Ansprüche, **dadurch gekennzeichnet, dass** der Halteansatz (3) als separates Folienelement hergestellt ist.

14. Vorrichtung (1) nach einem der vorhergehenden Ansprüche, **dadurch gekennzeichnet, dass** der Halteansatz (3) eine stutzen- oder konusartige, zum Ostium hin gerichtete oder auch in das Ostium oder Lumen hinein reichende Ausziehung (3b) aufweist, die eine einfache, passgenaue Fügung an die konzentrischen Schlauchlagen (2a,2b) des Ballonkorpus (2) ermöglicht.

15. Vorrichtung (1) nach einem der vorhergehenden Ansprüche, **dadurch gekennzeichnet, dass** der Halteansatz (3) mit der Ballonstruktur (2) verbunden ist, jedoch ohne Verwendung eines rigiden Verbindungs- oder Funktionskörpers, insbesondere wobei die Verbindung (2c) zwischen dem Ballonkorpus (2) und dem Halteansatz (3) durch Klebung oder Verschweißung hergestellt ist.

## Claims

1. A device (1) for a minimal irritating, optimal organ-tolerable sealing of a lumen (L), which opens as an ostium into the region of the body surface, in particular a natural or artificial anus (A) of a patient, comprising an extremely thin-walled, externally fillable balloon body (2) made of a soft-film-type material with a low volumetric expandability, which has at least one transostial region and a central lumen, which extends from the distal to the proximal end of the device (1) and serves for the reception of an insertion aid and/or allows the intestinal gas to escape, **characterized in that**
a) the central lumen inside the balloon body (2) is not surrounded by a shaft, so that rigid, potentially irritating components in the region of the ostium as well as in a possibly adjoining, extracorporeal region are entirely avoided, and **in that**
b) at least one flat, soft-film-type retaining projection (3) coated with a skin-tolerable adhesive adjoins to the proximal end of the balloon body (2) allowing a direct fixation of the device (1) to the skin of the patient.

2. The device (1) according to claim 1, **characterized in that** the balloon body (2) is entirely preformed to its required dimensions during manufacturing.

3. The device (1) according to claim 1 or 2, **characterized in that** the cavity of the balloon (2) is delimited towards the surrounding lumen by a layer of a soft-film-type material, especially wherein the layer of soft-film-type material delimiting the cavity of the balloon (2) towards the surrounding lumen is connected to or integrally formed with the outer envelope of the balloon body (2).

4. The device (1) according to one of the preceding claims, **characterized in that**, in order to produce the balloon body (2), one end (2a) of an initially tube-shaped balloon blank is inwardly everted and fed through the other end (2b) of the balloon blank, preferably wherein the two ends of the balloon (2a,2b) extending within each other are connected to each other in a tightly sealing manner, especially in the transostial or transluminal region or in the directly adjoining extracorporeal region, in order to produce an externally fillable space thereby, especially wherein the two ends of the balloon (2a,2b) extending within each other are directly connected to each other, i.e., not via a sleeve interposed therebetween or another spacing element.

5. The device (1) according to one of the preceding claims, **characterized in that** possibly existing, extracorporeal regions of the balloon body (2) are not radially expanded with respect to the transostial balloon section.

6. The device (1) according to one of the preceding claims, **characterized in that** the balloon body (2) is manufactured by blow molding, in particular by blow molding from a thermoplastic polyurethane film.

7. The device (1) according to one of the preceding claims, **characterized in that** the retaining projection (3) consists of a thin, preferably membrane-like soft film, and/or consists of thermoplastic polyurethane, and/or is manufactured by deep drawing.

8. The device (1) according to one of the preceding claims, **characterized in that** the retaining projection (3) is coated with a gel-like material layer, for example based on polyurethane.

9. The device (1) according to one of the preceding claims, **characterized in that** the retaining projection (3) comprises one or more lugs or wings projecting approximately radially away from the proximal end of the balloon (2a,2b), especially comprises a lug-like, wing-like or propeller-like base area.

10. The device (1) according to one of the preceding claims, **characterized in that** the retaining projection (3) has a symmetrical base area, in particular with two projections (16) lying approximately diametrically opposite to each other.

11. The device (1) according to one of the preceding claims, **characterized in that** the retaining projection (3) adjoins directly to the connecting region (2c) of the two ends (2a,2b) of the balloon film.

12. The device (1) according to one of the preceding claims, **characterized in that** the retaining projection (3) is attached or shaped as merging into one of the ends (2a,2b) of the balloon body (2).

13. The device (1) according to one of the preceding claims, **characterized in that** the retaining projection (3) is produced as a separate film element.

14. The device (1) according to one of the preceding claims, **characterized in that** the retaining projection (3) has a neck-like or cone-like extension (3b) oriented towards the ostium or even reaching into the ostium or lumen, which facilitates a simple, precisely fitting joining to the concentric tube layers (2a,2b) of the balloon body (2).

15. The device according to one of the preceding claims, **characterized in that** the retaining projection (3) is connected to the balloon structure (2), however, but without the use of a rigid connecting or functional body, especially wherein the connection (2c) between the balloon body (2) and the retaining projection (3) is produced by adhesion or welding.

## Revendications

1. Dispositif (1) de fermeture minimalement irritant, optimalement compatible avec les organes d'une lumière (L) débouchant dans la zone de la surface du corps sous forme d'ostium, en particulier d'un anus naturel ou artificiel (A) d'un patient, comprenant un corps de ballonnet (2) à paroi très mince, remplissable de l'extérieur, constitué d'un matériau de type feuille souple de faible extensibilité volumique, qui présente au moins une zone transostiale ainsi qu'une lumière centrale, qui s'étend de l'extrémité distale à l'extrémité proximale du dispositif (1) et qui sert à la réception d'un dispositif auxiliaire d'introduction et/ou qui permet l'évacuation de gaz intestinal, **caractérisé en ce que**
a) la lumière centrale à l'intérieur du corps de ballonnet (2) n'est pas entourée par une tige, de telle sorte que des composants rigides, ayant un effet potentiellement irritant sont totalement évités dans la zone de l'ostium ainsi que dans une éventuelle zone extracorporelle adjacente, et que
b) au moins un épaulement de retenue (3) plan, de type feuille souple, revêtu d'un adhésif non irritant pour la peau se raccorde à l'extrémité proximale du corps de ballonnet (2), qui permet une fixation directe du dispositif (1) sur la peau du patient.

2. Dispositif (1) selon la revendication 1, **caractérisé en ce que** le corps de ballonnet (2) est entièrement formé à la dimension requise lors de la fabrication.

3. Dispositif (1) selon la revendication 1 ou 2, **caractérisé en ce que** la cavité du ballonnet (2) est fermée en direction de la lumière entourée par une couche constituée d'un matériau de type feuille souple, en particulier **en ce que** la couche constituée d'un matériau de type feuille souple délimitant la cavité du ballonnet (2) en direction de la lumière entourée est reliée à l'enveloppe extérieure du corps de ballonnet (2) ou est fabriquée d'un seul tenant.

4. Dispositif (1) selon l'une des revendications précédentes, **caractérisé en ce que** pour la fabrication du corps de ballonnet (2) une extrémité (2a) d'une ébauche de ballonnet initialement tubulaire est retournée vers l'intérieur et est passée à travers l'autre extrémité (2b) de l'ébauche de ballonnet, de préférence **en ce que** les deux extrémités de ballonnet (2a, 2b) guidées l'une dans l'autre sont reliées l'une à l'autre de manière étanche, en particulier dans la zone transostiale ou transluminale ou dans la zone extracorporelle directement adjacente, afin de réaliser un espace remplissable de l'extérieur, en particulier **en ce que** les deux extrémités de ballonnet (2a, 2b) guidées l'une dans l'autre sont reliées directement l'une à l'autre, donc pas par l'intermédiaire d'un manchon inséré entre elles ou d'un autre écarteur.

5. Dispositif (1) selon l'une des revendications précédentes, **caractérisé en ce que** les zones extracorporelles éventuellement existantes du corps de ballonnet (2) ne sont pas étendues radialement par rapport à la section de ballonnet transostiale.

6. Dispositif (1) selon l'une des revendications précédentes, **caractérisé en ce que** le corps de ballonnet (2) est réalisé par moulage par soufflage, en particulier par moulage par soufflage à partir d'une feuille polyuréthane thermoplastique.

7. Dispositif (1) selon l'une des revendications précédentes, **caractérisé en ce que** l'épaulement de retenue (3) est constitué d'une feuille souple mince, de préférence de type membrane, et/ou est constitué de polyuréthane thermoplastique, et/ou est réalisé par emboutissage.

8. Dispositif (1) selon l'une des revendications précédentes, **caractérisé en ce que** l'épaulement de retenue (3) est revêtu d'une couche de matériau de type gel, par exemple à base de polyuréthane.

9. Dispositif (1) selon l'une des revendications précédentes, **caractérisé en ce que** l'épaulement de retenue (3) comporte une ou plusieurs pattes ou ailettes faisant saillie approximativement radialement de l'extrémité de ballonnet (2a, 2b) proximale, en particulier présente une surface de base en forme de patte, d'ailette ou d'hélice.

10. Dispositif (1) selon l'une des revendications précédentes, **caractérisé en ce que** l'épaulement de retenue (3) comporte une surface de base symétrique, en particulier avec deux prolongements (16) approximativement diamétralement opposés.

11. Dispositif (1) selon l'une des revendications précédentes, **caractérisé en ce que** l'épaulement de retenue (3) se raccorde directement à la zone de raccordement (2c) des deux extrémités (2a, 2b) de la feuille de ballonnet.

12. Dispositif (1) selon l'une des revendications précédentes, **caractérisé en ce que** l'épaulement de retenue (3) est moulé et/ou formé de manière à se prolonger dans l'une des extrémités (2a, 2b) du corps de ballonnet (2).

13. Dispositif (1) selon l'une des revendications précédentes, **caractérisé en ce que** l'épaulement de retenue (3) est réalisé sous forme d'élément de feuille séparé.

14. Dispositif (1) selon l'une des revendications précédentes, **caractérisé en ce que** l'épaulement de retenue (3) présente une partie étirée (3b) en forme de tubulure ou de cône, dirigée vers l'ostium ou également s'étendant dans l'ostium ou la lumière, qui permet un assemblage simple et précis avec les couches tubulaires (2a, 2b) concentriques du corps de ballonnet (2).

15. Dispositif (1) selon l'une des revendications précédentes, **caractérisé en ce que** l'épaulement de retenue (3) est reliée à la structure du ballonnet (2), toutefois sans utilisation d'un corps de raccordement ou fonctionnel rigide, en particulier **en ce que** le raccordement (2c) entre le corps de ballonnet (2) et l'épaulement de retenue (3) est réalisé par collage ou soudage.
